# EUROPEAN PATENT SPECIFICATION

(11) **EP 1 833 422 B1**
(45) Date of publication and mention of the grant of the patent: **16.11.2011**
(21) Application number: 05854323.2
(22) Date of filing: 16.12.2005
(51) Int. Cl.: A61F 2/16

(54) **PRELOADED IOL INJECTOR AND METHOD**
VORGELADENER IOL-INJEKTOR UND VERFAHREN
INJECTEUR DE LENTILLE INTRAOCULAIRE PRECHARGE ET SON PROCEDE

(30) Priority: 29.12.2004 US 25555
(43) Date of publication of application: 19.09.2007
(73) Proprietor: BAUSCH & LOMB INCORPORATED, Rochester, NY 14604 (US)
(72) Inventor: PYNSON, Joel, F-31400 Toulouse (FR); BESSIERE, Benoit, F-31280 Dremil Lafage (FR); RATHERT, Brian, D., Largo, FL 33778 (US)
(74) Representative: Vossius & Partner
(86) International application number: PCT/US2005/045577
(87) International publication number: WO 2006/071561

(56) References cited:
- EP-A- 0 519 282
- WO-A-2005/030097

## Description

### Background of the Invention

The present invention relates to ophthalmic surgical devices and methods. More particularly, the present invention relates to a device and method for inserting an intraocular lens (IOL) into an eye wherein the IOL may be conveniently preloaded in a component of the injector device.

IOLs are artificial lenses used to replace the natural crystalline lens of the eye when the natural lens has cataracts or is otherwise diseased. IOLs are also sometimes implanted into an eye to correct refractive errors of the eye in which case the natural lens may remain in the eye together with the implanted IOL. The IOL may be placed in either the posterior chamber or anterior chamber of the eye. IOLs come in a variety of configurations and materials. Some common IOL styles include the so-called open-looped haptics which include the three-piece type having an optic and two haptics attached to and extending from the optic; the one-piece type wherein the optic and haptics are integrally formed (e.g., by machining the optic and haptics together from a single block of material); and also the closed looped haptic IOLs. Yet a further style of IOL is called the plate haptic type wherein the haptics are configured as a flat plate extending from opposite sides of the optic. The IOL may be made from a variety of materials or combination of materials such as PMMA, silicone, hydrogels and silicone hydrogels, etc.

Various instruments and methods for implanting the IOL in the eye are known. In one method, the surgeon simply uses surgical forceps having opposing blades which are used to grasp the IOL and insert it through the incision into the eye. While this method is still practiced today, more and more surgeons are using more sophisticated IOL inserter devices which offer advantages such as affording the surgeon more control when inserting the IOL into the eye. IOL inserter devices have recently been developed with reduced diameter insertion tips which allow for a much smaller incision to be made in the cornea than is possible using forceps alone. Smaller incision sizes (e.g., less than about 3mm) are preferred over larger incisions (e.g., about 3.2 to 5+mm) since smaller incisions have been attributed to reduced post-surgical healing time and complications such as induced astigmatism.

Since IOLs are very small and delicate articles of manufacture, great care must be taken in their handling. In order for the IOL to fit through the smaller incisions, they need to be folded and/or compressed prior to entering the eye wherein they will assume their original unfolded/uncompressed shape. The IOL inserter device must therefore be designed in such a way as to permit the easy passage of the IOL through the device and into the eye, yet at the same time not damage the delicate IOL in any way. Should the IOL be damaged during delivery into the eye, the surgeon will most likely need to extract the damaged IOL from the eye and replace it with a new IOL, a highly undesirable surgical outcome.

Thus, as explained above, the IOL inserter device must be designed to permit easy passage of the IOL therethrough. It is equally important that the IOL be expelled from the tip of the IOL inserter device and into the eye in a predictable orientation and manner. Should the IOL be expelled from the tip too quickly or in the wrong orientation, the surgeon must further manipulate the IOL in the eye which could result in trauma to the surrounding tissues of the eye. It is therefore highly desirable to have an inserter device which allows for precise loading of the IOL into the inserter device and which will pass and expel the IOL from the inserter device tip and into the eye in a controlled, predictable and repeatable manner.

To ensure controlled expression of the IOL through the tip of the IOL inserter device, the IOL must first be loaded into the IOL inserter device. The loading of the IOL into the inserter device is therefore a precise and very important step in the process. Incorrect loading of an IOL into the inserter device is oftentimes cited as the reason for a failed IOL delivery sequence. Many IOL injector devices on the market today require the IOL to be loaded into the injector at the time of surgery by the attending nurse and/or surgeon. Due to the delicate nature of the IOL, there is a risk that the nurse and/or surgeon will inadvertently damage the IOL and/or incorrectly load the IOL into the injector device resulting in a failed implantation. Direct handling and/or loading of the IOL into the injector by the nurse and/or surgeon is therefore undesirable.

There remains a need for an IOL inserter and method which removes the need for direct handling of the IOL by the nurse and/or surgeon and which generally simplifies operation of the IOL injector device and IOL delivery process.

EP 0 519 282 A1 describes an intraocular lens injector having a holder with a bore holding the lens in a folded state. The holder is connected to a hollow cone. A plunger of the device inserted in the bore pushes the lens into and through the cone for controlled gradual injection into the eye and controlled gradual unfolding therein.

WO 2005/030097 A1, which represents prior art under Art. 54(3) EPC, describes a preloaded intraocular lens injector. The intraocular lens is positioned in a shuttle which is positioned inside a distal section of the device. At the time of use, the surgeon opens the vial and attaches a proximal section having a plunger to the distal section located in the vial. The proximal section is then lifted away from the vial together with the distal section, and the shuttle and the intraocular lens located in the distal section.

### Summary of the Invention

The present invention is defined by the features of the claims.

In a broad aspect of the invention, an injector device is provided having a proximal body section, a distal nozzle section, and a shuttle component wherein the IOL is loaded in an unstressed state which are packaged separate and then assembled together at the time of surgery. The injector device provides the IOL preloaded in the shuttle component of the device, preferably in an unstressed condition, i.e., in a condition where at least the IOL optic is not compressed or folded, and which is packaged either in a dry state or submersed in a vial of sterile storage solution (e.g., buffered saline). The package or vial is then sealed and sterilized. The solution maintains the IOL in a hydrated state during shipping and storage, a necessary requirement for IOLs made of certain materials such as hydrogels.

The proximal body section of the injector device is provided in a separately sealed and sterilized package although the proximal body section, nozzle section, and package or vial containing the shuttle component and IOL may be provided in a single "kit" type package if desired for sake of convenience to the user. The proximal body section of the injector device includes a tubular body having a longitudinal passageway extending between opposite, open ends thereof. A plunger component is inserted into the proximal open end of the tubular body and telescopes within the longitudinal passageway thereof. The plunger includes a finger press at the proximal end thereof for manually pressing and advancing the plunger through the passageway, and a plunger tip at the opposite, distal end thereof for engaging and pushing the IOL through and out the distal tip of the nozzle section of the injector device.

At the time of surgery, the nurse or surgeon simply opens the package containing these device sections and first attaches the nozzle section and shuttle component together. This first attachment step is preferably done inside the vial such that direct handling of the shuttle component is not required. A removable cover is placed in covering relation to the insertion tip portion of the nozzle section to permit manual handling of the nozzle section while the delicate insertion tip thereof remains covered and protected. The insertion tip is where the IOL is ultimately expelled from the injector device into an eye. Thus, the surgeon or nurse handles the nozzle section with the cover as the nozzle section is attached to the shuttle component. Once the nozzle and shuttle are coupled together, they are together withdrawn from the vial as a single unit. The shuttle and nozzle each include a longitudinal passageway which preferably lie along a common longitudinal axis when the shuttle and nozzle sections are assembled together. While still holding the cover, the nozzle section with shuttle is attached to the proximal body section of the injector device. Once the nozzle is attached to the body section, the cover is removed therefrom and the device is ready for injecting the IOL into a patient's eye.

The injector device includes means for compressing or otherwise urging the IOL into a smaller cross-section for delivery through the injector. In a preferred embodiment of the invention, the shuttle and nozzle passageways are configured with a narrowing taper towards the distal insertion tip. The plunger is advanced at the proximal end of the injector device causing the distal tip of the plunger to engage the IOL optic. As the plunger is advanced further, the IOL is pushed through the narrowing passageway, thereby compressing the IOL into a smaller cross-section and finally exiting at the distal end of the injector body and expressed into the eye in the intended manner.

The relative positioning of the IOL shuttle, the IOL and the injector device is such that upon attaching the proximal body section, nozzle and shuttle component of the injector device together, the IOL becomes preferentially positioned inside the injector device. The IOL thus becomes positioned in a particular orientation inside the injector device relative to the plunger tip. This IOL loaded position results in the leading haptic(s) being correctly aligned in the shuttle, and the trailing haptic(s) and optic aligning with the plunger tip so that upon advancement of the plunger, the plunger tip will engage the IOL optic in the intended manner without obstruction or jamming of the trailing haptic(s).

### Brief Description of the Drawings

Figure 1a is a side elevational view of an embodiment of the nozzle section approaching the vial to connect the nozzle section to the shuttle component contained within the vial;
Figure 1b is a side elevational view of an embodiment of the nozzle and shuttle component attached together and approaching the proximal body section of the injector device for connection thereto;
Figure 2a is a side elevational view of the fully assembled injector device with the protective sleeve being removed from the nozzle section;
Figure 2b is a perspective view of the fully assembled injector device of Figure 2a showing an IOL being expressed from the distal tip of the nozzle section thereof;
Figure 3a is an enlarged perspective view of the proximal body section of the injector device of Figures 2a,b;
Figure 3b is a side elevational view thereof;
Figure 3c is an end view thereof;
Figure 4a is a side elevational view of the plunger component of the injector device;
Figure 4b is a perspective view thereof;
Figures 5a is a perspective, top view of an embodiment of the shuttle component of the injector device with IOL loading area in the open position and an IOL positioned therein;
Figure 5b is a perspective view of the shuttle component of Figure 5a with the IOL loading area shown in the closed position and the shuttle being rotated 180o from the position shown in Figure 5a;
Figure 5c is a top plan view of the shuttle component of Figures 5a,b with the IOL loading area in the open position;
Figure 5d is an end view of Figure 5c taken from the left side thereof;
Figure 6a is a perspective view of the distal nozzle section of the injector device of the previous Figures;
Figure 6b is a top plan view thereof;
Figure 6c is a side elevational thereof;
Figure 6d is an end view thereof; and
Figure 7 is a perspective view of the distal nozzle section shown coupled to the shuttle component.

### Detailed Description

In a first, broad aspect, the invention comprises a preloaded injector device for injecting an IOL into an eye. The term "preloaded" as used herein means that a packaged component of the injector device includes an IOL positioned therein. Direct handling and loading of an IOL into the injector device is therefore not necessary.

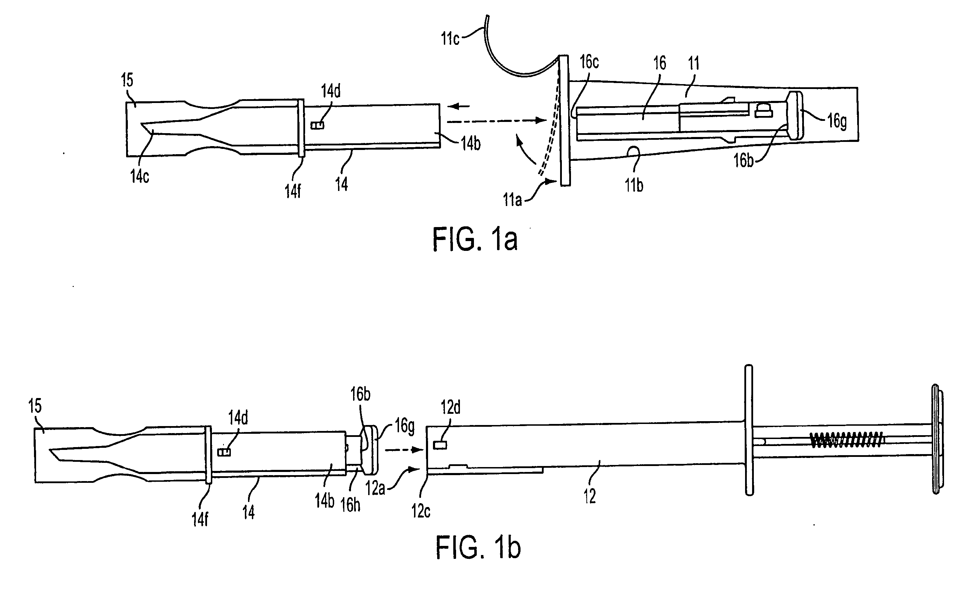

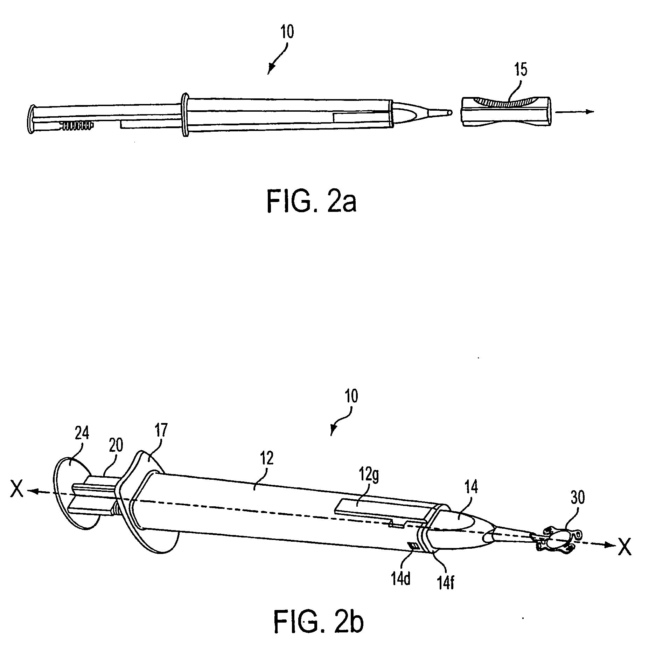

The steps to assemble the components of the injector device 10 are seen in Figs. 1a and b while the fully assembled injector device 10 is seen in Figs. 2a and b. Individual component parts of the injector device are shown in more detail in the remaining Figures. Injector device 10 includes a proximal body section 12, a distal nozzle section 14 and a shuttle component 16 which are packaged separately and then attached together at the time of surgery to ready the device for delivery of an IOL 30 therethrough and into a patient's eye (see Fig. 2b). The IOL 30 is preloaded into the shuttle component 16 of the device which is packaged in either a dry state or in a hydrated state in a container or vial 11 containing a storage solution to maintain the IOL in a hydrated state during shipping and storage. Whether the IOL is packaged and stored in the dry or wet state depends on the type of material from which the IOL is made. An example of an IOL material which may be packaged in the dry state is silicone while an IOL material which requires wet storage includes acrylic, for example.

The proximal body section 12 includes a longitudinal passageway 12a extending between the open proximal and distal ends 12b,12c thereof, respectively. The passageway 12a may assume any desired cross-sectional shape such as a rounded, rectangular shape as shown.

The distal nozzle section 14 includes a longitudinal passageway 14a extending between the open proximal end 14b and open distal tip 14c thereof. The passageway 14a tapers inwardly toward distal tip 14c so that the IOL is gradually compressed to a very small cross-section as it exits the device at tip 15c.

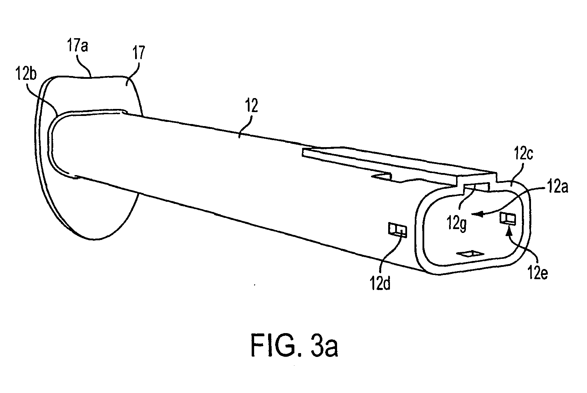

An exemplary embodiment of the shuttle component 16 is provided into which an IOL 30 is loaded and held in an uncompressed condition. This will be described in more detail below. During the first assembly step shown in Figs. 1a and b, shuttle 16, with an IOL 30 loaded therein, is positioned in distal nozzle section passageway 14a. Shuttle 16 also includes a longitudinal passageway 16a extending between the open proximal end 16b and open distal end 16c thereof. When shuttle 16 is positioned in distal nozzle section 14 the longitudinal passageways 16a, 14a of each are substantially aligned along the same axis X-X. When the proximal body section 12 is attached to the distal nozzle section 14, the longitudinal passageway 12a is substantially aligned along the common axis X-X of the distal and shuttle passageways 14a, 16a (Fig. 2b). It is noted that the shuttle 16 may be provided with a proximal flange 16q at proximal end 16b to assist in maintaining proper alignment between the proximal body section passageway 12a, plunger 20, and the shuttle 16. Flange 16q may or may not touch the inner wall surface defining proximal body section passageway 12a (see also Fig. 3a).

Referring again to proximal body section 12, a finger flange 17 may be formed anywhere along the length of body section 12, and preferably at the proximal end 12b thereof for ease in operating the injector device in the manner of a syringe. Finger flange is preferably configured with at least one straight edge 17a as shown (Fig. 3a) for resting device 10 on a flat surface. Other means may be provided for steadying the device on a flat surface such as, for example, making the body section 12 with one or more flat sides.

A plunger 20 having proximal and distal lengths 20a, 20b, respectively, a distal plunger tip 22, and a thumb press 24 telescopes within the proximal section 12. When the proximal and distal sections 12, 14 are attached together, the plunger 20 extends sequentially through proximal section passageway 12a and the shuttle passageway 16a so as to engage and push the IOL 30 through passageway 16a and out distal tip 15c. The IOL delivery sequence will be explained in more detail below.

It is understood that the overall configuration of the injector body 12 may vary from that shown and described herein. It is furthermore understood that the components of the injector device may be made of any suitable material (e.g., polypropylene) and may be wholly or partly opaque, transparent or translucent to better visualize the IOL within the injector device and the IOL delivery sequence. In a preferred embodiment of the injector device, the vial 11 and its contents are steam sterilized, requiring that the components, including the shuttle 16 located therein, are made from a material which can withstand the heat generated during steam sterilization. Examples of such materials include, but are not limited to, polypropylene, polycarbonate, polysulfone, ALTEM (by Dupont), and PFA. The injector body and distal nozzle section may be formed of a material that is sterilized with another method such as ETO sterilization, for example.

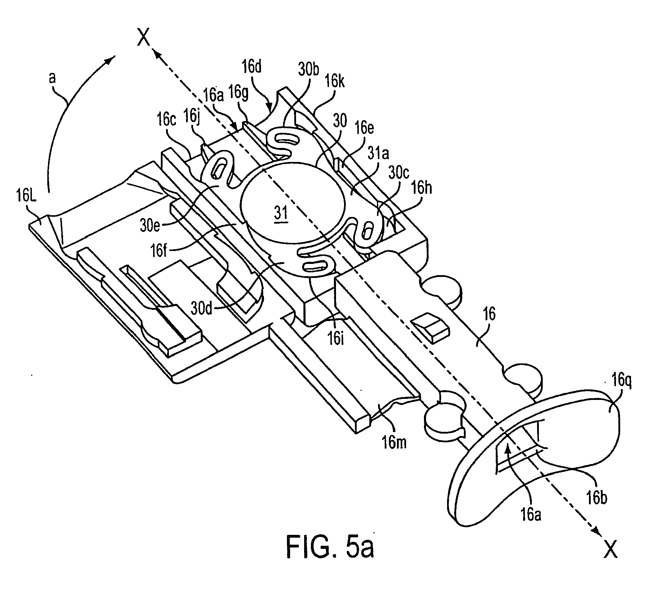

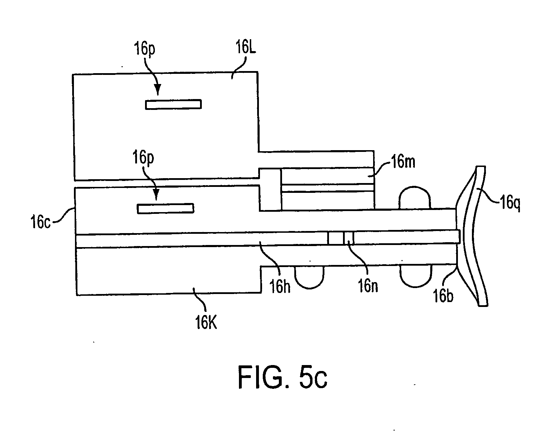

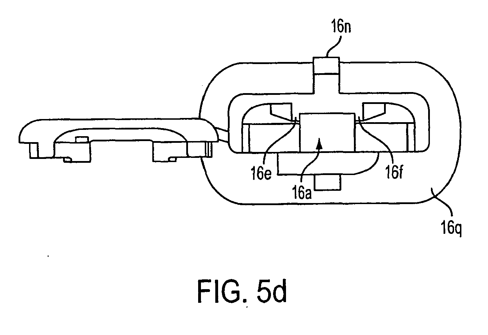

Shuttle component 16 is used for holding an IOL 30 in the preloaded position. The shuttle 16, with IOL 30 held thereby, is inserted into the distal nozzle section 14 through opening 14a thereof during the first assembly step seen in Fig. 1a. As seen best in Figures 5a-d, shuttle 16 includes an IOL loading area 16d wherein the IOL 30 is positioned in an unstressed state. Loading area 16d is in open communication with longitudinal passageway 16a and is configured to position the IOL 30 along axis X-X in an unstressed state and may include one or more optic support elements 16e,f each having a radius or other feature for aligning the IOL optic 31 along passageway 16a (and hence also axis X-X) about the periphery 31a thereof. Alternatively or in addition to the optic support elements, one or more haptic support elements 16g-j are provided on shuttle 16, each of which include a radius or other feature for aligning one or more haptics 30b-e which attach to and extend from the optic 31. In this regard, it is understood that the IOL configuration 30 shown and described herein is for discussion purposes only, and that the present invention is not to be limited thereby. The invention may be easily adapted to IOLs of any configuration and type (e.g., IOLs with plate, open or closed loop haptics, anterior chamber IOLs, posterior chamber IOLs, accommodating IOLs (including single and double lens types), etc.). The overall configuration of the IOL shuttle 16 and IOL loading area 16a may thus likewise vary so as to be cooperatively configured with and align the particular IOL style being used with the device. The shuttle 16 holds at least the IOL optic 31 in the unstressed state. It is furthermore preferable that shuttle 16 hold the IOL haptics at the correct vault angle (i.e., the angle from which they normally extend from the IOL optic periphery). It is even furthermore preferable that, in the case of an IOL having open looped haptics, the haptic support elements maintain the looped haptics at the correct angle and radius of curvature. In Fig. 5a, it is seen that the haptic support elements constrain the haptics along the outer curved edges thereof. This ensures that the haptic curvature, which is designed and set at manufacture of the haptics, does not increase or bend out of specification during storage of the IOL and shuttle.

At manufacture, the IOL 30 is placed in the shuttle 16. Positioning the IOL 30 in the shuttle 16 may be done by a worker using a pair of forceps, for example, although other methods may be used as desired, including automated or semi-automated means in an assembly line. To facilitate loading of the IOL in the shuttle, the IOL loading area 16a may be formed with two wall sections 16k and 16L which are pivotally connected (e.g., via a living hinge 16m) to enable opening and closing of the IOL loading area 16d. Wall sections 16k and 16L are spread open in a coplanar relationship in the open position of the shuttle loading area 16d. In this open position, IOL loading area 16d is easily accessible and an IOL 30 may be simply placed upon one of the two sections, preferably upon section 16k. This may be done by aligning the IOL optic 31 with the IOL supporting elements 16g,j and aligning the haptics 30b-e with the haptic support elements 16d, 16e, respectively.

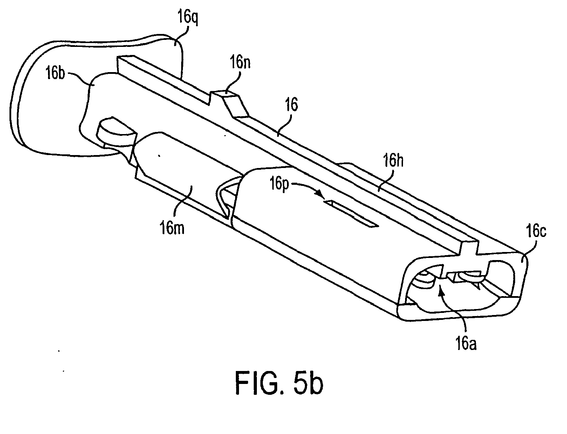

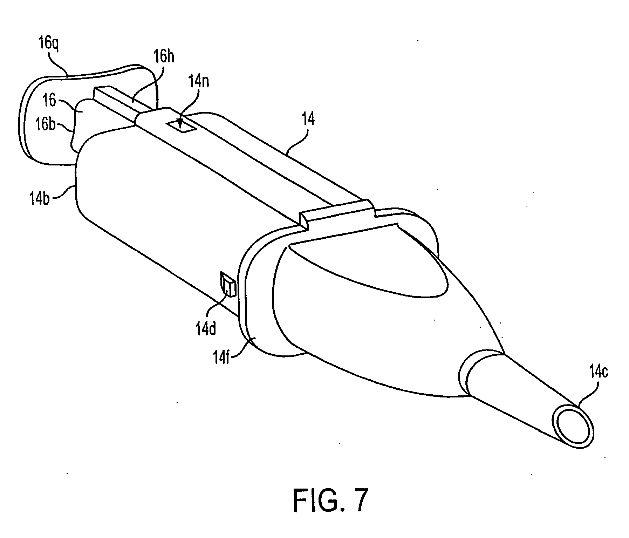

Once the IOL 30 is properly positioned in the shuttle IOL loading area 16a, the two sections 16g, 16h are pivoted together (in the direction of arrow "a" in Fig. 5a) to the closed position which encases IOL 30 between the now facing wall sections 16k, 16L (Fig. 5b). With the IOL 30 thus positioned in the shuttle 16 and the shuttle wall sections 16k, 16L closed, shuttle 16 is inserted into the vial 11 for storage. For wet packaging, to ensure storage solution reaches the IOL 30, the shuttle may include one or more through-holes 16p which are in open communication with the IOL 30. One of many possible embodiments of a vial is seen in Figure 1a wherein a vial 11 having an open end 11a and an internal cavity 11b is provided to accept the shuttle 16 with the shuttle distal end 16c thereof lying adjacent the open end 11a of the vial. One or more longitudinally extending fins or other alignment features (not shown) may be formed on the inside surface of vial 11 to align and maintain the shuttle 16 at the desired orientation within vial 11. A rigid cover or a flexible cover sheet such as a foil seal 11c is attached to open end 11a to seal the vial. Seal 11c may be tethered to vial 11 by a flexible hinge (not shown) if desired. This feature keeps the seal with the vial after vial opening and thereby prevents having a "loose" part in the operating suite. At the time of surgery, the package or vial 11 is removed from any outer packaging (e.g. a TYVEC pouch) in a sterile field and the vial cover seal 11c is removed to open vial 11 and access shuttle 16 therein. The proximal body section 12 and distal nozzle section 14 are likewise removed from their packaging in a sterile field. The surgeon or nurse then inserts the proximal end 14b of the distal nozzle section 14 into the vial 11 to couple the shuttle component 16 thereto. As seen in Figs. 1a and 1b, a protective sleeve 15 is optionally provided in covering relation to at least the insertion tip 14c of nozzle section 14. Sleeve 15 allows manual handling of the nozzle 14 while protecting the tip 14c from damage during handling and assembling of the injector device. Once the injector device 10 is fully assembled, the sleeve is removed from the nozzle 14 as explained below. The sleeve 15 may be formed of a suitable plastic such as polycarbonate or plypropylene, for example. When fully coupled together, the nozzle and shuttle are removed from the vial as a single unit with the proximal end 16b of the shuttle preferably although not necessarily extending outwardly of the proximal end 14b of the distal nozzle section as seen in Figs. 1b and 7.

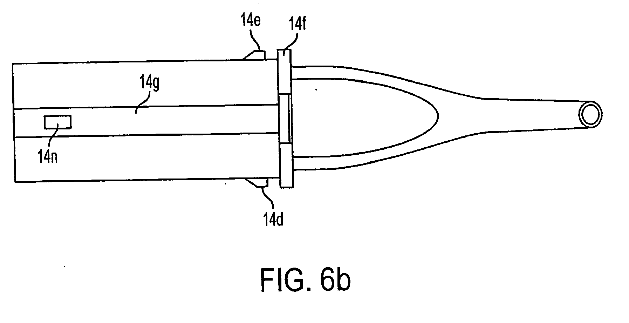

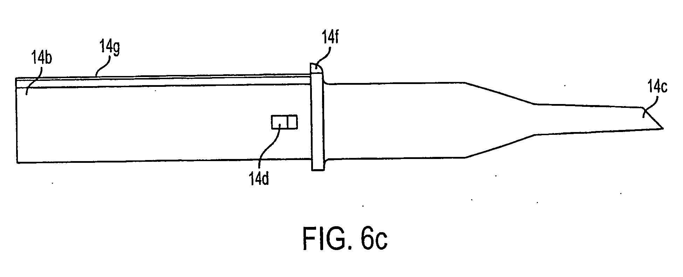

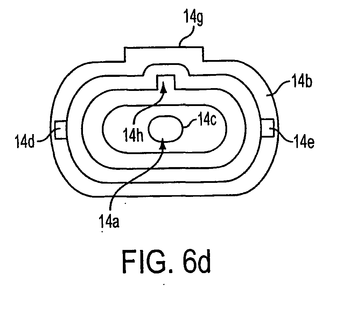

To assist in attaching the shuttle 16 to the distal nozzle section 14 in the correct manner, a longitudinal groove 14h (Fig. 6d) may be formed on an inner wall surface of distal nozzle section 14 which aligns with a longitudinal flange 16h formed on an outer wall surface of shuttle 16 (Fig. 5b). As such, the shuttle 16 may be slidingly received within distal nozzle section 14 with groove 14h and flange 16h providing a "key" to prevent incorrect coupling between the shuttle and distal section. Furthermore, the shuttle 16 and distal nozzle section 14 may be fixed in the assembled condition through suitable mechanical locking features. For example, the shuttle 16 may be provided with a detent 16n and the distal nozzle section 14 provided with a slot 14n which engage upon full advancement of the shuttle 16 within the distal nozzle section 14. It will thus be realized that the shuttle 16 thus becomes fixed to the distal nozzle section 14.

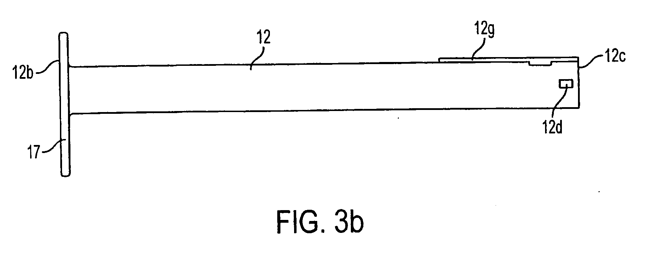

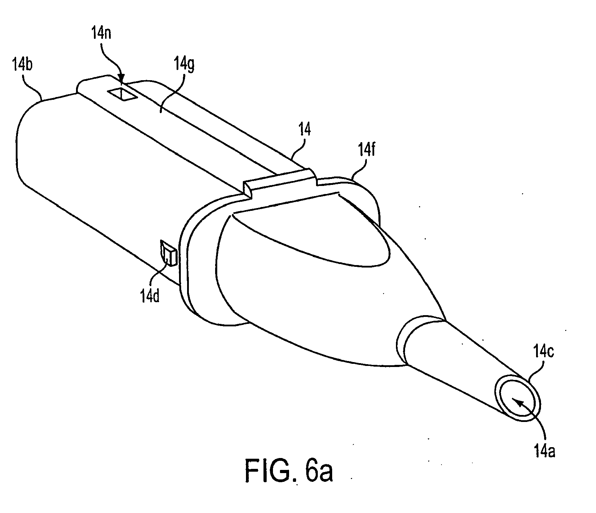

Once the nozzle section and shuttle have been coupled together and removed from vial 11, the nurse or surgeon attaches the nozzle section 14 to the proximal body section 12. Various mechanical connection features may be employed to permit the quick and easy attachment of the proximal body section 12 to the distal nozzle section 14 by simply pressing the two sections together as described above. Such features may include cooperating detents and recesses or a friction fit between the two sections, for example. In the embodiment shown in the Figures, a pair of detents 14d,e (Figs. 6a-d) are provided on the outer wall surface of distal section 14 which align with and engage a pair of through-holes 12d,e formed on proximal body section 12 adjacent open distal end 12c thereof (Figs. 3a,b). When the proximal body section 12 and distal nozzle section 14 are brought together, the detents 14d,e engage the through-holes 12d,e, respectively, and the sections become attached together. A radial flange 14f may be provided on distal nozzle section 14 to act as a stop against further advancement of the proximal section 12 on the distal section 14, i.e., to prevent advancement beyond the point of detent engagement

Once the proximal body and distal nozzle sections are attached together, the cover 15 may be removed from nozzle section 12 as seen in Fig. 2a. The assembly of the injector device is now complete and the surgeon may proceed to inject the IOL 30 into a patient's eye by inserting nozzle tip 14c into an incision formed in the eye and pressing plunger 20 to advance the IOL 30 through and out the nozzle tip 14c (see Fig. 2b; the eye not shown for sake of clarity).

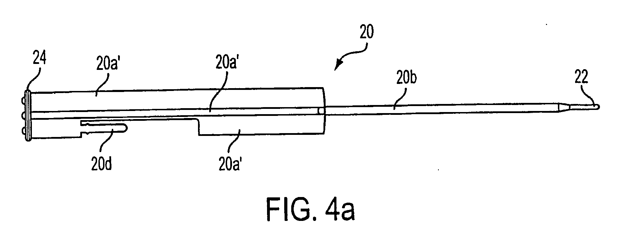

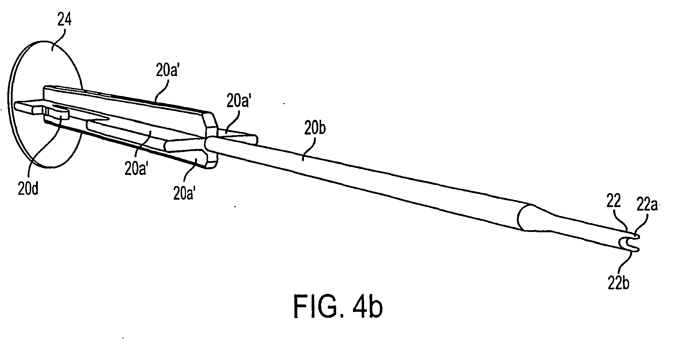

Referring to Figures 4a,b, it is seen that the plunger 20 includes distal and proximal plunger shaft lengths 20a, 20b, respectively, having a plunger tip 22 at the distal end thereof and a thumb press 24 at the proximal end thereof for manually operating the injector device. The plunger tip 22 is configured for engaging the IOL optic 31 at the periphery 31a thereof as the plunger 20 is advanced toward the distal tip 14c of distal section 14. It is very important that the plunger tip 22 not damage the IOL optic 31. The plunger tip 22 is thus designed to prevent damage to the IOL optic 31. In one possible embodiment, the tip is bifurcated into first and second tip portions 22a and 22b, whereby the IOL optic periphery 31a becomes engaged between tip portions 22a, 22b as seen in Figure 2B. It is understood that other plunger tip designs may be used with the present invention as desired and the invention is not limited by the plunger style. It is furthermore preferred that the plunger shaft is rotationally fixed within passageway 12a to prevent unexpected rotation of the shaft (and thus the tip 22) therein. For example, the plunger shaft may be rotationally fixed by forming the proximal shaft length 20b and passageway 12a non-circular in cross-section as shown.

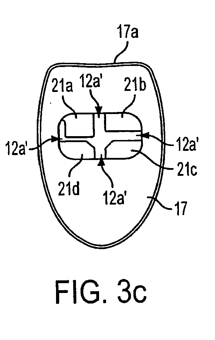

In a particularly advantageous embodiment, the proximal length 20b of the plunger shaft is provided with one or more elongated flanges 20a' which align with a like number of slots 12a' formed between radially extending fins 21 a-d formed on the inner wall surfaces of proximal section 12 adjacent proximal end 12b thereof (Fig. 3c). The purpose of flanges 20a' and slots 12a' is to provide tactile resistance therebetween and thereby allowing the surgeon more precise control and feel when advancing the plunger. The fins 21 a-d may be made rigid or flexible, or a combination thereof, to provide the amount of tactile resistance desired. It is understood that other ways of providing plunger control and tactile resistance between the plunger and injector body are within the scope of this invention. This provides the surgeon with continuous tactile feedback allowing the surgeon to advance the plunger (and thus the IOL) through the injector device in a very concise and controlled manner. Additionally, the flanges 20a' and slots 12a' help provide proper centering of the plunger shaft 20 and tip 22 relative to axis X-X along which the passageways of the components lie as explained above. Upon full advancement of the plunger, it is desirable to have the plunger automatically retract to some degree upon release of finger pressure against plunger finger press 24. In this regard, a spring 20c may be provided on a finger 20d on shaft length 20a. As the plunger is advanced, the spring 20c will interact with the one or more of the fins 21a-d as the plunger 20 is advanced therethrough.

When it is time to use the injector device, the surgeon selects a container or vial 11 having the appropriate IOL style and power preloaded in the shuttle 16 stored in the vial 11 as described above. The outer packaging is removed in a sterile field of the surgical suite. The distal nozzle section 14 and proximal body section 12 having the plunger 20 coupled thereto is also removed from its associated packaging in the sterile filed. The nurse or surgeon then attaches the proximal nozzle section 12 to the shuttle 16 and removes it from the vial, and then attaches the nozzle/shuttle unit to the proximal body section in the manner described above. The surgeon may then insert the distal tip 14c into an incision cut into the eye and begin advancing the plunger 20. As the plunger 20 is advanced, the plunger tip 22 enters shuttle passageway 16a, engages the optic periphery 31a and pushes IOL 30 forwardly. Upon continued advancement of the plunger 20, the IOL 30 is pushed through the shuttle passageway 16a and is expressed from distal tip 14c and into the eye (Fig. 2b). As stated above, the spring 20c provides increasing bias in the reverse direction as the plunger reaches the fully advanced position. This occurs as spring 20c is compressed against one or more of the fins 21a-d. This assists the surgeon in maintaining precise control over plunger (and hence IOL) advancement and allows automatic retraction of the plunger upon relieving the pushing pressure being exerted against the plunger thumb press 24. This is useful for easily executing a second stroke of the plunger in order to engage and manipulate the trailing haptic into place in the eye. This feature, together with the bifurcated plunger tip 22, allows a more precise control and manipulation of the IOL with the plunger tip in-situ than would be possible with an injector device not having these features.

As discussed above, the device may be used for IOLs of any type and style. The configuration of the various component parts may likewise vary to accommodate the particular IOL style being employed with the device. It may thus be realized that the present invention provides an injector device method and apparatus that may be provided in a variety of embodiments. The present invention is therefore not to be limited by the embodiments shown and described herein.

## Claims

1. A method of packaging an intraocular lens (30) in a portion of an intraocular lens injection device (10) comprising the steps of:
a) providing a distal nozzle section (14);
b) placing a sleeve (15) in removable, covering relation to at least a portion of said distal nozzle section (14) and placing said distal nozzle section and sleeve in a package;
c) providing a shuttle (16) and positioning the intraocular lens (30) therein; and
d) depositing said shuttle and intraocular lens in a vial (11) containing storage solution and sealing the vial.

2. The method of claim 1. and further comprising the steps of:
a) providing a proximal body section (12) and a plunger (20) slidably received in said proximal body section (12);
b) opening the vial (11) and attaching the distal nozzle section (14) to the shuttle (16) and removing the distal nozzle section (14), shuttle (16) and intraocular lens (30) together from the vial (11), and
c) attaching the distal nozzle section (14) to the proximal body section (12), whereby said intraocular injection device is ready for injecting the intraocular lens (30) into an eye.

3. A method of preparing an injector device for use, said injector device reconfigurable from a storage condition to an injection condition, the injector device comprising a) a proximal body section (12) having a longitudinal passageway (12a); b) a distal nozzle section (14) having a longitudinal passageway (14a); and c) a shuttle (16) having a longitudinal passageway (16a) with an intraocular lens (30) positioned therein,
the shuttle (16) and the intraocular lens (30) being positioned and sealed in a vial (11) having an open end (11a) leading into an internal cavity (11b)
said method comprising the steps of:
a) opening said vial (11);
b) attaching said distal nozzle section (14) to said shuttle (16) when the shuttle is disposed in the vial; and
c) lifting said distal nozzle section away from said vial and thereby removing said distal section (14), said shuttle (16) and said intraocular lens (30) from said vial.

4. An injector device assembly (10) reconfigurable from a storage condition to an injection condition, said injector device assembly comprising:
a) a proximal body section (12) having a first longitudinal passageway (12a);
b) a distal nozzle section (14) having a second longitudinal passageway (14a) and a sleeve in removable, covering relation to at least a portion of said distal nozzle section;
c) a shuttle (16) having a third longitudinal passageway (16a), said shuttle (16) holding an intraocular lens (30) therein; and
d) a vial (11) having an open end leading into an internal cavity and a closure for removably sealing said open end, said shuttle being positioned and sealed in said vial (11) and said distal nozzle section (14) being configured to receive said shuttle (16) into the second longitudinal passageway (14a) and said proximal body section (12) being configured to receive said distal section in the first longitudinal passageway (12a).

5. The device of claim 4, wherein each of said proximal body section (12), said distal nozzle section (14) and said shuttle (16) is configured such that when the shuttle (16) is received into the second longitudinal passageway (14a) and said distal nozzle section (14) is received in the first longitudinal passageway (12a), each of the first longitudinal passageway (12a), the second longitudinal passageway (14a) and the third longitudinal passageway (16a) lie substantially along a common axis.

6. The device of claim 5 wherein the first longitudinal passageway (12a), the second longitudinal passageway (14a) and the third longitudinal passageway (16a) are coaxially arranged.

7. The device of claim 4 wherein said shuttle (16) and said distal section are configured to be snap fit together.

## Patentansprüche

1. Verfahren zum Verpacken einer Intraokularlinse (30) in einem Teil einer Intraokularlinseninjektionsvorrichtung (10), das die Schritte aufweist:
a) Bereitstellen eines distalen Düsenabschnitts (14);
b) Anordnen einer Hülse (15) in einer entfernbaren Abdeckbeziehung mit mindestens einem Teil des distalen Düsenabschnitts (14) und Anordnen des distalen Düsenabschnitts und der Hülse in einer Verpackung;
c) Bereitstellen eines Shuttles (16) und Anordnen der Intraokularlinse (30) darin; und
d) Anordnen des Shuttles und der Intraokularlinse in einer Ampulle (11), die eine Lagerflüssigkeit enthält, und Versiegeln der Ampulle.

2. Verfahren nach Anspruch 1, das ferner die Schritte aufweist:
a) Bereitstellen eines proximalen Körperabschnitts (12) und eines verschiebbar in den proximalen Körperabschnitt (12) aufgenommenen Kolbens (20);
b) Öffnen der Ampulle (11) und Befestigen des distalen Düsenabschnitts (14) am Shuttle (16) und Entfernen des distalen Düsenabschnitts (14), des Shuttles (16) und der Intraokularlinse (30) zusammen aus der Ampulle (11); und
c) Befestigen des distalen Düsenabschnitts (14) am proximalen Körperabschnitt (12), wodurch die Intraokularinjektionsvorrichtung bereit ist, die Intraokularlinse (30) in ein Auge zu injizieren.

3. Verfahren zum Vorbereiten einer Injektorvorrichtung auf den Gebrauch, wobei die Injektorvorrichtung von einem Lagerzustand zu einem Injektionszustand umkonfigurierbar ist, wobei die Injektorvorrichtung aufweist: a) einen proximalen Körperabschnitt (12) mit einem longitudinalen Durchgang (12a); b) einen distalen Düsenabschnitt (14) mit einem longitudinalen Durchgang (14a); und c) ein Shuttle (16) mit einem longitudinalen Durchgang (16a) mit einer darin angeordneten Intraokularlinse (30),
wobei das Shuttle (16) und die Intraokularlinse (30) in einer Ampulle (11) angeordnet und versiegelt sind, die ein offenes Ende (11a) aufweist, das in einen inneren Hohlraum (11b) führt;
wobei das Verfahren die Schritte aufweist:
a) Öffnen der Ampulle (11);
b) Befestigen des distalen Düsenabschnitts (14) am Shuttle (16), während das Shuttle in der Ampulle angeordnet ist; und
c) Abheben des distalen Düsenabschnitts von der Ampulle und dadurch Entfernen des distalen Abschnitts (14), des Shuttles (16) und der Intraokularlinse (30) aus der Ampulle.

4. Injektorvorrichtungsanordnung (10), die von einem Lagerzustand zu einem Injektionszustand umkonfigurierbar ist, wobei die Injektorvorrichtungsanordnung aufweist:
a) einen proximalen Körperabschnitt (12) mit einem ersten longitudinalen Durchgang (12a);
b) einen distalen Düsenabschnitt (14) mit einem zweiten longitudinalen Durchgang (14a) und einer Hülse in einer entfernbaren Abdeckbeziehung mit mindestens einem Teil des distalen Düsenabschnitts;
c) ein Shuttle (16) mit einem dritten longitudinalen Durchgang (16a), wobei das Shuttle (16) eine Intraokularlinse (30) darin hält; und
d) eine Ampulle (11), die ein offenes Ende, das in einen inneren Hohlraum führt, und einen Verschluss zum entfernbaren Versiegeln des offenen Endes aufweist, wobei das Shuttle in der Ampulle (11) angeordnet und versiegelt ist und der distale Düsenabschnitt (14) konfiguriert ist, das Shuttle (16) in den zweiten longitudinalen Durchgang (14a) aufzunehmen, und der proximale Körperabschnitt (12) konfiguriert ist, den distalen Abschnitt in den ersten longitudinalen Durchgang (12a) aufzunehmen.

5. Vorrichtung nach Anspruch 4, wobei der proximale Körperabschnitt (12), der distale Düsenabschnitts (14) und das Shuttle (16) jeweils so konfiguriert sind, dass wenn das Shuttle (16) in den zweiten longitudinalen Durchgang (14a) aufgenommen wird und der distale Düsenabschnitt (14) in den ersten longitudinalen Durchgang (12a) aufgenommen wird, der erste longitudinale Durchgang (12a), der zweite longitudinale Durchgang (14a) und der dritte longitudinale Durchgang (16a) jeweils im wesentlichen längs einer gemeinsamen Achse verlaufen.

6. Vorrichtung nach Anspruch 5, wobei der erste longitudinale Durchgang (12a), der zweite longitudinale Durchgang (14a) und der dritte longitudinale Durchgang (16a) koaxial angeordnet sind.

7. Vorrichtung nach Anspruch 4, wobei das Shuttle (16) und der distale Abschnitt konfiguriert sind, ineinander einzurasten.

## Revendications

1. Procédé de conditionnement d'une lentille intraoculaire (30) dans une partie d'un dispositif d'injection de lentille intraoculaire (10) comprenant les étapes consistant à :
a) fournir une section de buse distale (14) ;
b) placer une gaine (15) dans une relation de recouvrement amovible par rapport à au moins une partie de ladite section de buse distale (14) et placer lesdites section de buse distale et gaine dans un boîtier ;
c) fournir une navette (16) et positionner la lentille intraoculaire (30) dans celle-ci ; et
d) déposer lesdites navette et lentille intraoculaire dans une fiole (11) contenant une solution de stockage et fermer hermétiquement la fiole.

2. Procédé selon la revendication 1, et comprenant en outre les étapes consistant à :
a) fournir une section de corps proximale (12) et un piston (20) reçu de manière coulissante dans ladite section de corps proximale (12);
b) ouvrir la fiole (11) et fixer la section de buse distale (14) à la navette (16) et retirer la section de buse distale (14), la navette (16) et la lentille intraoculaire (30), ensemble, de la fiole (11) ; et
c) fixer la section de buse distale (14) à la section de corps proximale (12), moyennant quoi ledit dispositif d'injection intraoculaire est prêt pour injecter la lentille intraoculaire (30) dans un oeil.

3. Procédé de préparation d'un dispositif d'injection pour une utilisation, ledit dispositif d'injection pouvant être reconfiguré d'une condition de stockage dans une condition d'injection, le dispositif d'injection comprenant: a) une section de corps proximale (12) comportant un passage longitudinal (12a) ; b) une section de buse distale (14) comportant un passage longitudinal (14a) ; et c) une navette (16) comportant un passage longitudinal (16a), une lentille intraoculaire (30) étant positionnée à l'intérieur de celui-ci,
la navette (16) et la lentille intraoculaire (30) étant positionnées et enfermées hermétiquement dans une fiole (11) comportant une extrémité ouverte (11a) menant dans une cavité interne (11b),
ledit procédé comprenant les étapes consistant à :
a) ouvrir ladite fiole (11) ;
b) fixer ladite section de buse distale (14) à ladite navette (16) lorsque la navette est disposée dans la fiole ; et
c) élever ladite section de buse distale hors de ladite fiole et, de ce fait, retirer ladite section distale (14), ladite navette (16) et ladite lentille intraoculaire (30) de ladite fiole.

4. Ensemble de dispositif d'injection (10) pouvant être reconfiguré d'une condition de stockage dans une condition d'injection, ledit ensemble de dispositif d'injection comprenant :
a) une section de corps proximale (12) comportant un premier passage longitudinal (12a) ;
b) une section de buse distale (14) comportant un deuxième passage longitudinal (14a) et une gaine dans une relation de recouvrement amovible par rapport à au moins une partie de ladite section de buse distale ;
c) une navette (16) comportant un troisième passage longitudinal (16a), ladite navette (16) contenant une lentille intraoculaire (30) dans celle-ci ; et
d) une fiole (11) comportant une extrémité ouverte menant dans une cavité interne et une fermeture pour fermer hermétiquement de manière amovible ladite extrémité ouverte, ladite navette étant positionnée et enfermée hermétiquement dans ladite fiole (11) et ladite section de buse distale (14) étant configurée pour recevoir ladite navette (16) dans le deuxième passage longitudinal (14a) et ladite section de corps proximale (12) étant configurée pour recevoir ladite section distale dans le premier passage longitudinal (12a).

5. Dispositif selon la revendication 4, dans lequel chacune de ladite section de corps proximale (12), de ladite section de buse distale (14) et de ladite navette (16) est configurée de sorte que, lorsque la navette (16) est reçue dans le deuxième passage longitudinal (14a) et que ladite section de buse distale (14) est reçue dans le premier passage longitudinal (12a), le premier passage longitudinal (12a), le deuxième passage longitudinal (14a) et le troisième passage longitudinal (16a) s'étendent chacun sensiblement le long d'un axe commun.

6. Dispositif selon la revendication 5, dans lequel le premier passage longitudinal (12a), le deuxième passage longitudinal (14a) et le troisième passage longitudinal (16a) sont agencés coaxialement.

7. Dispositif selon la revendication 4, dans lequel ladite navette (16) et ladite section distale sont configurées pour être assemblées par pression.
